(19) Europäisches Patentamt / European Patent Office / Office européen des brevets

(11) **EP 3 308 803 B1**

(12) **EUROPEAN PATENT SPECIFICATION**

(45) Date of publication and mention
of the grant of the patent:
**03.08.2022 Bulletin 2022/31**

(21) Application number: **17194995.1**

(22) Date of filing: **05.10.2017**

(51) International Patent Classification (IPC):
**A61L 2/07** $^{(2006.01)}$   **G01N 21/31** $^{(2006.01)}$
**G01N 21/15** $^{(2006.01)}$   **A61L 2/28** $^{(2006.01)}$

(52) Cooperative Patent Classification (CPC):
**A61L 2/07; A61L 2/28; G01N 21/31;** A61L 2202/14;
A61L 2202/24; G01N 2021/151

(54) **STERILIZER APPARATUS WITH MEASUREMENT CHAMBER HAVING ORIFICE AND CORRESPONDING METHOD**

STERILISATORVORRICHTUNG MIT MESSKAMMER MIT ÖFFNUNG UND ZUGEHÖRIGES VERFAHREN

APPAREIL DE STÉRILISATION AVEC CHAMBRE DE MESURE COMPORTANT UN ORIFICE ET PROCÉDÉ CORRESPONDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.10.2016 NL 2017617**

(43) Date of publication of application:
**18.04.2018 Bulletin 2018/16**

(73) Proprietor: **Miele & Cie. KG**
**33332 Gütersloh (DE)**

(72) Inventors:
• **Kopinga, Klaas**
**5506 Veldhoven (NL)**

• **Van Doornmalen, Josephus Paulus Clemens Maria**
**5688 Oirschot (NL)**

(56) References cited:
**DE-U1-202014 009 495     US-A- 4 507 875**

• **Jpcm Doornmalen - Gomez Hoyos ET AL: "Surface steam sterilization : steam penetration in narrow channels", , 1 January 2013 (2013-01-01), XP055388492, DOI: 10.6100/IR758412 ISBN: 978-90-38-63442-5 Retrieved from the Internet: URL:https://pure.tue.nl/ws/files/3573650/758412.pdf [retrieved on 2017-07-06]**

**Description**

**Field of the invention**

The present invention relates to a device and method to test and monitor steam sterilization conditions, in particular surface steam sterilization conditions, in real-time. The invention further relates to a sterilizer apparatus.

**Background art**

Conditions for surface steam sterilization are predetermined time-temperature relations when saturated steam is present, e.g., 3 minutes at 134 °C with saturated steam [MRC59]. These surface steam sterilization conditions are derived from sterilization of aqueous liquids, in which the mechanism for the killing of organisms is coagulation of proteins [SYK67]. Sterilization is achieved if the liquid is kept at a certain elevated temperature for a sufficient amount of time. In the literature several temperature-time combinations for sterilization have been documented. The results for aqueous liquids can be used for surface steam sterilization [MRC59] if the steam heats up all surfaces to be sterilized to the required temperature and forms condensate on these surfaces. In the standard EN285 [EN285] this has been translated to the requirement that steam should have direct contact with the surfaces and the supplied steam may contain only a very small amount of non-condensable gases (NCGs) (3.5 vol. %, relative to the condensate, which corresponds to approximately 0.006 vol. % in the vapor phase). With NCGs in the steam gases are meant that will not condense in the pressure and temperature range of steam sterilization. These ranges are 0 to 350 kPa and 0 to 150 °C, respectively. The NCGs can be introduced by or originate from the environmental air or dissolved gases in the feed water for the steam generation. Examples of NCGs originating from the environment are a bad air removal before the sterilization phase, leaks in the sterilizer or the connected devices, such as a vacuum pump, and compressed air injected into the sterilizer by leaks in valves controlled by compressed air (typically 700 kPa) and gaskets.

When sterilizing medical (surgical) instruments, all surfaces that can be in contact with the environmental air have to be sterile. This also may involve the inside surfaces of instruments. Since the 1990s Minimal Invasive Surgery (MIS) is developing fast. Instruments for MIS often contain narrow channels. Also the inside of these channels have to be exposed to sterilization conditions. Therefore steam penetration is important and not trivial for surface steam sterilization of this type of instruments [VDO13-1,VDO13-3,VDO15-1,VDO15-2].

Steam sterilizers can contain various components, e.g., mechanical, electronic and software components. These components can malfunction unexpectedly. Also fluctuations in a steam supply can occur, for example, fluctuations in the amount of NCGs in the feed water for steam generation. The importance of ensuring a correct steam sterilization process is acknowledged in the standards for steam sterilization [EN285, EN13060, ISO17665], patents [US5270217] and literature [VDO13-2]. This demonstrates that monitoring is 'good practice' for steam sterilization processes. Moreover, according to these standards it is mandatory to monitor each load, e.g., ISO 17665 clause 10.1 specifies: 'Routine monitoring and control shall be performed on each operating cycle.' Currently, monitoring in steam sterilization is based on Biological, Chemical and Physical indicators.

In order to test if all surfaces are exposed to steam sterilization conditions a steam penetration test has to be performed each day before starting production with a steam sterilizer, e.g., ISO 17665 clause 12.1.6 states: 'If the sterilization process relies on the removal of air from the sterilizer chamber in order to achieve rapid and even penetration of steam into the sterilizer load, a steam penetration test shall be carried out each day before the sterilizer is used'. The current golden standard for steam penetration tests is based on a textile towel pack [B&D63]. Over the years this test is further specified in the standards [ISO11140-3] and alternative steam penetration tests [ISO11140-4, ISO11140-5]. Also test packs have been reduced in size. Presently, it is questioned in the field and standard committees if a textile towel pack can be used to test steam penetration in channels of medical devices. Therefore other Process Challenge Devices (PCD) have been developed. For instance, channels with one end open and at the other end a chamber / receptacle to accommodate an indicator are used [EN867-5, EN13060].

Clause 8.2.4 of the standard EN 285 [EN285] specifies an air detector. This device is based on a tube that is connected to the steam sterilizer chamber at one end whereas the other end, outside of the steam sterilizer, is closed. The temperature at the closed end of the tube is measured with a temperature sensor. This air detector has only limited value, as already demonstrated in the note of clause 13.3.1 [EN285]: 'NOTE This method does not necessarily express the true content of NCG in steam. The limiting value was defined experimentally in the 1960s in relation to the sensitivity of air detectors commonly used in the UK at that time. Repeated measurements give an idea of the true picture of NCGs in the steam supply.' Furthermore the device has to be calibrated against a standard textile towel pack [EN285, ISO11140-3, IS011140-4]. Also, because it qualifies conditions based on temperature measurements only, it cannot qualify steam concentration or the presence of air (NCGs). Finally, it is a channel with one end closed, of which the initial conditions are not specified and therefore not known. Therefore it is unclear what actually will be measured and where the energy for the temperature increase is coming from.

Steam quality (the amount of NCGs present in the steam) and the capacity for steam penetration of a sterilizer can vary over the day. In many hospitals worldwide Minimal Invasive Surgery (MIS) instruments with narrow channels are sterilized. The steam quality might even be insufficient to establish steam sterilization in these channels.

Biological monitoring is performed with biological indicators (BIs). These indicators have the disadvantage that their integrity can only be guaranteed if the storage and handling is performed according to the specifications given by the manufacturer. Apart from this, the incubation of the indicator will inevitably take time. Consequently, release of loads after sterilization will be delayed until the results of the indicators are available. Apart from this, their disadvantage is that they only provide a one sided test. This implies that in case of a problem, the information will often be insufficient for trouble shooting.

Chemical monitoring is performed with chemical indicators (CIs). Like the BIs, also these indicators only provide a one-sided test. One of the drawbacks of a CI is that it is rather inaccurate [VDO12-2] and many CIs have to be judged by subjective color interpretation [US4115068]. Additionally, the CI can only be interpreted after the complete sterilization cycle has been performed and the CI is taken out of the load. Also the CI is often intended to mimic a biological killing mechanism. The correspondence of the relatively simple chemical reactions with the complex biological killing mechanism is not obvious.

Currently, physical monitoring is mostly performed by monitoring pressure and temperature. From the pressure the so-called 'theoretical temperature' is calculated [EN285]. In the known method, the theoretical temperature is compared to the measured temperature. If the difference is less than 2 K the steam is accepted as saturated steam. However, according to the literature [IAWPS] this assumption is not correct. The calculation of the theoretical temperature is based on the pressure-temperature relation for saturated steam and, consequently, only valid when saturated steam is present. Only measuring pressure and temperature is not sufficient to ensure sterilization conditions [VDO14-1].

In some cases it is tried to measure if the steam injected into the steam sterilizer chamber is saturated. If that is the case the pressure-temperature for saturated steam is used again. However, even if this method would be accurate enough, insufficiently removed initial air and leaks in the sterilizer chamber will not be detected. Consequently, steam sterilization conditions cannot be ensured by measuring the steam quality in the supply line of steam (outside of the sterilizer chamber), e.g., the 'SteamSpy' from MMM GmbH, Germany [DE102010016017A1].

According to some standards [EN285] a steam penetration test has to be performed before starting production with a steam sterilizer. Currently, a Bowie and Dick towel pack [B&D63, ISO11140-3, ISO11140-4] is the golden standard for the steam penetration tests. This test is based on chemical indicators. This leads to subjective human interpretation of the results in daily practice. Also many alternative tests claiming to meet the standard for alternative tests [ISO11140-4] appear to not fulfil their claims [KIRK12]. A second drawback is that the steam penetration in a textile towel pack (porous material) is quite different from the steam penetration in modern medical instruments with narrow channels used in (minimal invasive) surgery.

In some standards hollow Process Challenge Device (PCDs) are specified [EN867-5, EN13060]. In the literature these devices are discussed and proven to not fulfil their claims [ESEN11-1, ESEN12-1], basically because of three reasons. First, the volume of the receptacle for the BI or CI at the end of the tube enhances the steam penetration compared to a regular tube, which may lead to false passes [VDO15-2]. Second, for most chemical indicators the color (change) has to be interpreted by subjective human vision [VDO12-2]. In case of a BI incubation time is necessary. Third, during production the PCDs are used constantly. In between successive sterilization cycles the devices are not conditioned. This implies that the initial conditions before use can vary, causing a less reproducible and reliable final result.

Finally, physical or electronic steam penetration tests are available in the market. It is reported that 3 out of 4 commercially available steam penetration test do not fulfil the claimed standard [BEN11]. Patents of the tested devices are for the EBI 15, Ebro Electronic GmbH, Ingolstadt, Germany, [DE202006006926U1] (The newer version EBI 16 is based on similar principles), and the DPCD-3, Digital Process Challenge Device version 3 professional, Interster International BV, Wormerveer, the Netherlands, [EP1230936A1]. One device is based on the time derivative of the temperature [VDO13-3]. This is the 3M™ Electronic Test System (ETS), 3M Deutschland GmbH, Neuss, Germany [WO201047139]. This device should only be used as a steam penetration test as specified in the ISO 11140 part 4 [ISO11140-4]. Because the ISO 11140-4 defines that such a penetration test is only to be performed once a day before starting the production [ISO17665], this device is designed to use it only once a day. Also it is calibrated to mimic the results of a textile towel pack [ISO 11140-4], which makes it unsuitable to judge steam penetration in hollow instruments. Finally, like all biological and chemical monitoring products mentioned so far, it requires manual handling of human operators of sterilizers. This costs time in a production environment and has the risk of human errors. Other methods that are discussed in patents are heat sinks to identify NCGs [US6323031].

The document "Surface steam sterilization: Steam penetration in narrow channels", van Doornmalen - Gomez Hoyos, ISBN: 978-90-38-63442-5, and the DE 202014009495 U1 respectively, show a sterilizer apparatus comprising a sterilizer chamber, wherein the sterilizer apparatus is configured to inject steam into the sterilizer chamber, a measurement probe comprising a measurement chamber having a fluid connection to the sterilizer chamber, a light source configured to emit light into the measurement chamber, and a light detector configured to detect the light in the measurement chamber and convert the detected light into a detection signal.

The US 4507875 A describes a device for determining the concentration of condensable vapor in a flowing gas stream.

**Summary of the invention**

The present invention seeks to provide an improved device to measure in particular steam sterilization conditions.

According to an aspect of the invention, a sterilizer apparatus is provided. The sterilizer apparatus comprises:

a sterilizer chamber, wherein the sterilizer apparatus is configured to inject steam into the sterilizer chamber;
a measurement probe comprising a measurement chamber having a fluid connection to the sterilizer chamber;
a light source configured to emit light into the measurement chamber;
a light detector configured to detect the light in the measurement chamber and convert the detected light into a detection signal;
wherein the measurement chamber comprises an orifice for supply of a gas.

The orifice allows to clean the measurement chamber by supplying the gas through the orifice. This may improve the accuracy of the measurement result of the measurement probe.

The sterilizer apparatus is configured to perform a sterilizing process to sterilize a load, and to automatically blow the gas through the orifice into the measurement chamber before or after the sterilizing procedure. This way, the measurement chamber is cleaned before or after the sterilizing procedure. This may improve the accuracy of the measurement, as any leftover fluids may be flushed away out of the measurement chamber before the next sterilization process is started. For example, the sterilizer may be configured to automatically, for example under control of a controller or processor, perform a process including the sterilizing process and the blowing of the gas through the orifice. As part of the sterilizing process, the sterilizer apparatus may, in a particular example, further be configured to evacuate the sterilizer chamber before injecting the steam or to perform evacuation of sterilizer chamber and injection of the steam alternatingly.

The orifice and the fluid connection with the sterilizer chamber are located on opposite sides of the measurement chamber. This way, the gas blown through the orifice into the measurement chamber blows any fluids into and through the fluid connection, into the sterilizer chamber.

The controller may be configured to determine an indication of a vapor density or whether a liquid is present in the measurement chamber based on the detection signal. This determination may be achieved by comparing the detection signal to one or more reference signals, for example. This determination becomes more accurate and more reproducible if any fluids are flushed out of the measurement chamber and fluid connection thereof before a measurement session is started.

The controller may be configured to, repeatedly during a sterilization process performed by the sterilizer apparatus, receive the detection signal from the light detector and determine the indication of the vapor density or whether the liquid is present based on the detection signal. This allows to follow the changes of the conditions within the measurement chamber during the sterilization process. Preferably, to mimic the conditions in other objects to be sterilized, the flushing operation is not performed in between these measurements.

The steam sterilizer may be configured to adjust the sterilization process based on the indication of the vapor density or whether the liquid is present. This helps to carry out a successful sterilization by adapting the sterilization process based on the measurements.

In an embodiment, a capillary tube is fitted to the orifice to fluidly connect the measurement chamber to a source of the dry gas. A capillary tube, due to its small diameter, does not alter the properties of the measurement chamber too much, making it unnecessary to provide a closing valve to close the orifice.

The apparatus may comprise a valve to selectively block or allow the passage of dry air from the source of the dry air into the capillary tube and into the measurement chamber. The valve may be positioned at the end of the capillary tube, which may be easier to manufacture.

The fluid connection to the sterilizer chamber may comprise a resistance such as a tube, wherein a first end of the resistance has a fluid connection to the measurement chamber and a second end of the resistance is open. This helps to mimic the conditions inside tubular instruments that are to be sterilized. These conditions may thus be replicated inside the measurement chamber.

The condensation may comprise condensation of water vapor. The measurement probe may be specially designed to detect condensation of water vapor by properly selecting light frequencies and intensity thresholds for the detected light intensity, specific for the presence of condensation of water vapor on a surface in the measurement chamber.

The light source may comprise at least one of a lamp and a light guide. The light source may comprise both a lamp and a light guide. The light guide may be configured to guide the light from the light source to the measurement chamber and towards the surface.

The light detector may comprise at least one of a light sensor and a light guide. The light detector may comprise both the light sensor and the light guide. The light guide may be configured to guide the light reflected from or transmitted through the surface towards and onto the light sensor. To this end, the light guides of the light source and light detector are positioned so that these light guides cooperate with each other to form a light path through the measurement chamber from the light source via the surface to the light detector.

According to another aspect of the invention, a method of sterilizing a load is provided in claim 8.

According to an aspect of the invention, due to the variations of vapor conditions inside sterilization devices during the day, it may be advantageous to monitor every load using a reproducible and absolute method.

The person skilled in the art will understand that the features described above may be combined in any way deemed useful. Moreover, modifications and variations described in respect of an apparatus may likewise be applied to a corresponding method, and modifications and variations described in respect of the method may likewise be applied to the apparatus.

## Brief description of the drawings

The present invention will be discussed in more detail below, with reference to the attached drawings. Throughout the drawings, similar items may be indicated by means of the same reference numerals. It is noted that the drawings are not on scale.

Fig. 1A illustrates a first example of a vapor density or surface condensation measurement probe using transmission mode detection.

Fig. 1B illustrates a first example of vapor density or surface condensation measurement probe using reflection mode detection.

Fig. 2 illustrates a device for monitoring steam sterilization conditions.

Fig. 3A illustrates a second example of a vapor density or surface condensation measurement probe using transmission mode detection.

Fig. 3B illustrates a second example of a vapor density or surface condensation measurement probe using reflection mode detection.

Fig. 4 illustrates a third example of a vapor density or surface condensation measurement probe.

Fig. 5 illustrates a fourth example of a vapor density or surface condensation measurement probe.

Fig. 6 illustrates some examples of locations of the measurement probes within a sterilizer chamber or at the wall of the sterilizer chamber.

Fig. 7 shows a flowchart illustrating a method of sterilizing a load.

Fig. 8 shows a flowchart illustrating another method of sterilizing a load.

Fig. 9 shows a flowchart illustrating a method of detecting condensation.

## Detailed description

In the IR wavelength region between roughly 1000 and 1600 nm, the optical absorption of water (vapor) shows various strong peaks, whereas the absorption of air and other non-condensable gases is smaller by several orders of magnitude. Therefore, these wavelengths are used in the following description in a device to detect water and water vapor. However, it will be understood that the principles disclosed herein may also be used in conjunction with other wavelengths, including visible light and ultraviolet light, as desired. In addition, other radiation types may be used and their absorption, transmission, and/or reflectance may be measured to detect properties of the available water (vapor), using the principles set forth herein.

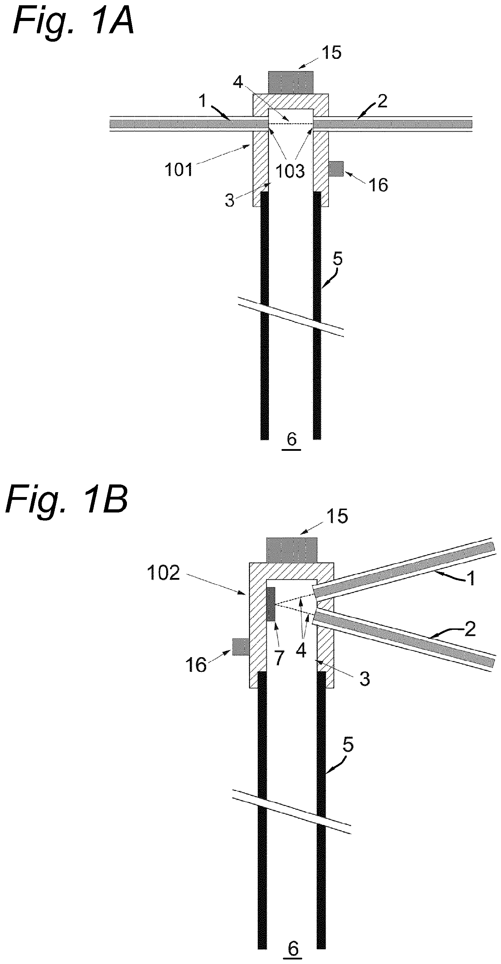

A probe 101 to measure the water vapor density or the surface condensation is schematically depicted in Fig. 1A. An alternative implementation of a measurement probe 102 is schematically depicted in Fig. 1 B.

The probe 101 comprises a measurement chamber 3. The walls of the measurement chamber 3 can, for example, be made of a material that is resistant to saturated steam at elevated temperatures (i.e., in the applied temperature range, for example between 100 and 150 degrees Celsius), is impermeable to gases and fluids, optionally has a thermal conductivity that is substantially higher than that of an air-vapor mixture, and is optically nontransparent. One of the materials having these properties is stainless steel, in particular stainless steel grade 316L, which has the additional advantage that its surface can be polished to create optical mirrors.

The inside of the measurement chamber can have a cylindrical shape. Such a cylindrical shape mimics the channels used in MIS instruments and is relatively easy to manufacture.

Typical inner dimensions of the measurement chamber range between a few mm and a few cm. Criteria that may be taken into account for determining the inner diameter of the measurement chamber include the diameter of the channel of a medical instrument that may be mimicked and the desired optical path length. Typically, the measurement chamber is much smaller than the sterilizer chamber, for example a length or width of the measurement chamber may be smaller than a tenth or a hundredth of the length, width, or depth of the sterilizer chamber.

The light emitted by a suitable IR source, for instance a light emitting diode (LED), enters a measurement chamber 3 via a first optical fiber 1 and leaves the chamber via a second optical fiber 2, by which the light is guided to an IR detector. The IR absorption by the water vapor is measured along the optical path 4. The absorption that occurs along the optical path 4 depends on the density of the water vapor and the optical path length within the measurement chamber. Some possible arrangements of the measuring chamber are illustrated in Fig. 1. In the arrangement of Fig. 1A, the light emitted by the first optical fiber 1 is directly collected by the second optical fiber 2. In the arrangement shown in Fig. 1B, a reflective surface 7, e.g. a mirror, is provided. The reflective surface 7 may optionally be concave, for example, to direct more of the light into the second optical fiber 2. The light emitted by the first optical fiber 1 is reflected by the reflective surface 7 and collected by the second optical fiber 2. Compared to the arrangement shown in Fig. 1A, the arrangement shown in Fig. 1B has a longer optical path 4, because the light travels through the measurement chamber 3 two times.

In both the arrangements of Fig. 1A and Fig. 1B, an optional tube 5 can be fitted to the measurement chamber 3. This way, a Process Challenge Device can be formed.

The connection between the tube 5 and the measurement chamber 3 should be leak tight, which can be realized, for example, by welding both parts together, if the tube 5 is also made of metal. Alternatively, for instance, a polytetrafluoroethylene (PTFE) or synthetic rubber (e.g. Viton) O-ring seal can be used that is compressed between the tube 5 and a groove in the measurement chamber 3 by means of a screw mechanism (not shown in the illustrations). This arrangement has the advantage that the tube 5 can be exchanged by another tube, and hence tubes with a different length or different properties can be used, if desired. Apart from this, such a seal enables the use of tubes 5 made of other materials, such as polymers. A similar seal can be used to fix the optical fiber to the measurement chamber, although many alternative feedthroughs are possible.

The inner diameter and the length of the tube 5 can be chosen to reflect the properties (e.g. dimensions) of the channels present in MIS instruments, which are to be sterilized. In a typical case this inner diameter can be in the order of a few mm. However, the precise diameter of the tube 5 appears to be not very critical. The length of the channel may be a more important parameter to replicate steam penetration condition within the MIS instrument that is to be sterilized. Depending on the channels to be sterilized, the length of the 'challenge tube' can be identified and chosen. The length of the Process Challenge Device may be chosen approximately equal to the length of the channel that is to be sterilized. Alternatively, for safety, the length of the Process Challenge Device may be selected to be longer than the channel to be sterilized. In case there are multiple channels to be sterilized with different lengths, the Process Challenge Device can have a tube length based on the longest channel that is to be sterilized. Via the open end 6 of the tube 5 the air-vapor mixture in the sterilizer chamber can enter the Process Challenge Device. At the closed end of the tube 5, where the measurement chamber 3 is fitted, the vapor conditions created are thus made similar to the vapor conditions inside the channels present in the MIS instruments, which are to be sterilized.

For example, the inner diameter of the measurement chamber 3 can be chosen equal (or substantially equal) to the inner diameter of the tube 5. This avoids the above-mentioned complications induced by the receptacle for BI's or CI's present in standard hollow PCD's [VDO15-2].

To withstand the harsh conditions within the sterilizer chamber, the optical fibers can be made of a glass-glass type with a protective coating of, for instance, polyimide. Multimode fibers with a core diameter in the range 200 - 800 $\mu$m may be suitable for this purpose. The metal parts of the measurement chamber can be made of stainless steel (for example 316L) or another material that is not eroded, for example, by saturated steam.

Suitable optical path lengths for these arrangements can be between 2 and 15 mm. However, the path length is not a limitation of the present invention.

For water vapor at a pressure of 3 bar the IR absorption for a wavelength of 1450 nm - one of the absorption peaks - is of the order of a few percent. This would make the system rather inaccurate and prone to instabilities of the optical system.

In an embodiment, two different wavelengths may be used to increase the measurement accuracy. Two wavelengths may be used, which have different absorption properties. For example, wavelengths of approximately 1300 and 1450 nm have such different absorption properties. By using two wavelengths with different absorption properties, the accuracy may be largely improved and the instabilities in the optical system are suppressed effectively [VDO13-1]. For example, the two wavelengths may be implemented by time multiplexing the light emitted by two LEDs into one optical fiber and using the same detector for both wavelengths. However, other arrangements are also possible, for example using separate pairs of optical fibers for each wavelength or using the same optical fiber for both the incoming and reflected IR signal.

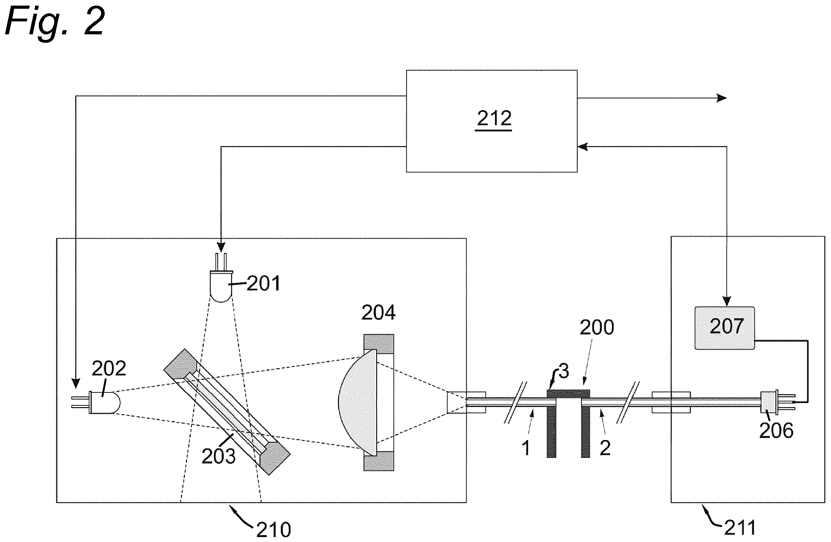

An example realization of the measurement system, including a transmitter 210 and a receiver 211, is schematically depicted in Fig. 2. The transmitter 210 comprises two light emitting diodes LED1 201 and LED2 202, a combiner 203, and a lens 204. The receiver 211 comprises a detector 206 and an analog to digital converter (ADC) 207. The transmitter 210 and the receiver 211 are optically coupled to the measurement chamber 3 by means of a first optical fiber 1 and a second optical fiber 2, respectively. In operation, the LEDs 201, 202 are switched on and off alternatingly. The light from both LEDs 201, 202 is coupled into an optical fiber 1 via a combiner 203 and a lens 204. The light passing through the optical fiber 1 is then coupled into the measurement chamber 3. Light from inside the measurement chamber 3 is guided by the second optical fiber 2 towards the detector 206 of the receiver 211. The optical signal transmitted through the fibers 1, 2 and the measurement chamber 3 is converted by the detector 206 into an analog voltage that is supplied to the analog to digital converter (ADC) 207. The ADC 207 is configured to output a digital value corresponding to the voltage supplied to it.

If the optical absorption $\alpha$ in the measurement chamber is of the order of a few percent, the output signals $V_1$ and $V_2$ of the ADC for LED1 and LED2, respectively, vary linear with the absorption and are given by:

$$V_1 = I_1 \times \eta_{LED1} \times S(\lambda_1) \times [1 - \rho \times d \times f(\lambda_1)] \times \eta_{DET}(\lambda_1) \times G_{ADC}$$

$$V_2 = I_2 \times \eta_{LED2} \times S(\lambda_2) \times [1 - \rho \times d \times f(\lambda_2)] \times \eta_{DET}(\lambda_2) \times G_{ADC}$$

In these equations $I_1$ and $I_2$ denote the current through LED1 201 and LED2 202, respectively, and $\eta_{LED1}$ and $\eta_{LED2}$ the efficiency of LED1 201 and LED2 202, respectively. The transmission of the optical fiber system (combiner 203, lens 204, fibers 1, 2, etc.) is denoted by S and may (slightly) depend on the wavelength $\lambda$ of the IR light. The steam density in the measurement chamber is denoted by p, whereas d denotes the optical path length within the measurement chamber 3. The function f reflects the specific absorption of water vapor, which depends strongly on the wavelength $\lambda$ of the light. The efficiency of the detector is denoted by $\eta_{DET}$ and also depends on the wavelength $\lambda$. The gain of the ADC, including its preamplifier, is denoted by $G_{ADC}$. It will be understood that these equations are only disclosed here as a working example. Other equations and formulas may be used to process the measured values, based on the principles disclosed herein.

In the absence of water vapor the IR absorption is negligible, and in that case the equations above may be simplified to

$$V_1 = I_1 \times \eta_{LED1} \times S(\lambda_1) \times \eta_{DET}(\lambda_1) \times G_{ADC}$$

$$V_2 = I_2 \times \eta_{LED2} \times S(\lambda_2) \times \eta_{DET}(\lambda_2) \times G_{ADC}$$

The voltages $V_1$ and $V_2$ may be made equal to each other by adjusting the current $I_1$ supplied to LED1 201 and the current $I_2$ supplied to LED2 202. The value of $V_1$ and $V_2$ in the absence of water vapor is denoted hereinafter by $V_0$. Thus, in absence of water vapor, it is assumed that $V_1 = V_2 = V_0$. If water vapor is present, the values of $V_1$ and $V_2$ can be expressed as

$$V_1 = V_0 \times [1 - \rho \times d \times f(\lambda_1)]$$

$$V_2 = V_0 \times [1 - \rho \times d \times f(\lambda_2)]$$

The difference signal $V_\rho = V_1 - V_2$ is given by

$$V_\rho = V_0 \times \rho \times d \times [f(\lambda_1) - f(\lambda_2)]$$

By dividing the difference voltage $V_\rho$ by $V_0$ one obtains

$$V_\rho = \rho \times d \times [f(\lambda_1) - f(\lambda_2)] = C_{cal} \times \rho$$

Here $C_{cal}$ is a calibration constant, which only contains the absorption properties of water vapor and the optical path length in the measurement chamber. Herein, $C_{cal}$ is defined as

$$C_{cal} = d \times [f(\lambda_1) - f(\lambda_2)]$$

In a particular example implementation, if the LEDs are alternatingly switched on and off at a rate of 14 kHz, a 12 bit ADC is used, the signals $V_1$ and $V_2$ are sampled at 56 kHz, and the result is averaged over 0.25 s, the absorption signal $V_\rho$ can be measured with a precision better than 1% of the value corresponding to saturated steam. These parameters are merely examples.

It is important to realize that the determination of the vapor density may be influenced by several additional effects.

First, if an air-vapor mixture is present in the measurement chamber, droplet formation may occur, resulting in Rayleigh scattering and Mie scattering of the IR light. Depending on the arrangement of the measurement chamber this will lead to a decrease or an increase of the light intensity reaching the detector, and can lead to large errors in the inferred values of the vapor density.

Second, the absorption peaks of fluid water are shifted with respect to those of water vapor.

Third, any surface condensation occurring within the measurement chamber may dramatically affect the transmission or reflection of the IR light, precluding a reliable measurement of the vapor density.

These effects can be almost completely eliminated by heating the components of the measurement chamber to a temperature sufficiently (e.g., a few degrees) above the temperature of the steam entering the sterilizer chamber. This can be realized either by an optional dedicated heater 15 and temperature control unit or by thermally linking the measurement chamber to a specific part of the sterilizer which has a temperature that is sufficiently high. Such a sufficiently warm part of the sterilizer can be, e.g., a heated jacket of the sterilizer that is controlled in such a way that its temperature is higher than the sterilizer chamber temperature. Thus, the specific part of the sterilizer is a part that has facilities to heat it to a temperature that is higher than the remainder of the sterilizer, in particular a temperature that is higher than the temperature of the steam that is injected into the sterilizer chamber.

In a specific example of a possible realization of the device according to Fig. 1A and Fig. 2 the light sources 201 and 202 may be implemented by Light Emitting Diodes that emit light of around 1300 nm and 1450 nm, respectively. These LEDs may be alternatingly switched on and off at a frequency of, for example, 14 kHz by currents in the range of 20 to 40 mA, yielding about 2 mW of optical output in the wavelength range around 1300 nm and 1450 nm. The light of these two sources may be combined and focused into the fiber 1 using a polka dot beam splitter, for example, as the combiner 203. An aspheric lens, for example, may be used for the lens 204.

Using this configuration, for example, about 0.2 mW of optical power of each LED may be effectively coupled into fiber 1. For fibers 1 and 2, for example a glass-glass fiber with a polyimide coating may be used, which will withstand temperatures in the used temperature range. An optical fiber with a numerical aperture of 0.26 may be used. The full acceptance angle for light may be about 30°. A fiber with a core diameter of 0.6 mm may be used. Connections between the different fibers and optical components may be implemented using SMA (SubMiniature version A) connectors. Using the configuration thus described, the power of the light entering the measurement chamber 3 may be about 0.1 mW.

The length of the optical path within the measurement chamber may be, for example, 7 mm. This implies that about 2% of the incident light (2 $\mu$W) is collected by fiber 2. In a particular example, effectively 1 $\mu$W of light reaches the detector 206 when the measurement chamber 3 contains environmental air. As detector 206 a photodiode may be used. This photodiode may have a sensitive region with a diameter of, for example, 1 mm and a sensitivity of, for example, 0.9 A/W. The light receiver 211 may further comprise a transconductance amplifier (not shown in the drawing) configured to convert the detector signal into a voltage. This transconductance amplifier may comprise an operational amplifier. For example, the amplifier may yield an output signal of 200 mV/$\mu$W and an RMS noise of the order of 100 $\mu$V at a bandwidth of 400 kHz. This results in a signal to noise ratio of 300 (peak-to-peak noise), which may be improved by over a factor of 100 by sampling and averaging the signal over a given time interval.

In such an embodiment, the optical signals at 1300 nm and 1450 nm may decrease by 2% and 4%, respectively, when saturated steam at 134 °C (3.14 bar) is present in the measurement chamber (compared to environmental air). The above-described equations may be used to program the controller to determine the concentration of water vapor. In an alternative embodiment, the controller 212 may be configured to compare the intensity of the received optical signals to a predetermined threshold, to determine whether the concentration of water vapor satisfies a corresponding predetermined concentration. A look-up table may be provided to map detected light intensities to pre-calculated corresponding water vapor concentrations.

The occurrence of condensation on the tips of fibers 1 and 2 may enhance these percentages of reduction of detected light intensities by about a factor of 2. When using a configuration as shown in Fig. 1B (reflection geometry), condensation occurring on both the fiber tips and the reflective surface 7 was found to decrease the output signal at 1450 nm by over 20% in an experiment. The use of additional sheets or lenses 8 (explained hereinafter in greater detail) also causes the intensity of the detected optical signals to be decreased by a particular percentage when condensation forms on the sheets or lenses. The amount of decrease of light intensity depends on the specific implementation and may be determined by experiment. Thus, to detect condensation, a threshold may be derived from these percentages and used to program the controller 212. When the detected light intensity drops below the predetermined threshold, condensation is detected by the controller.

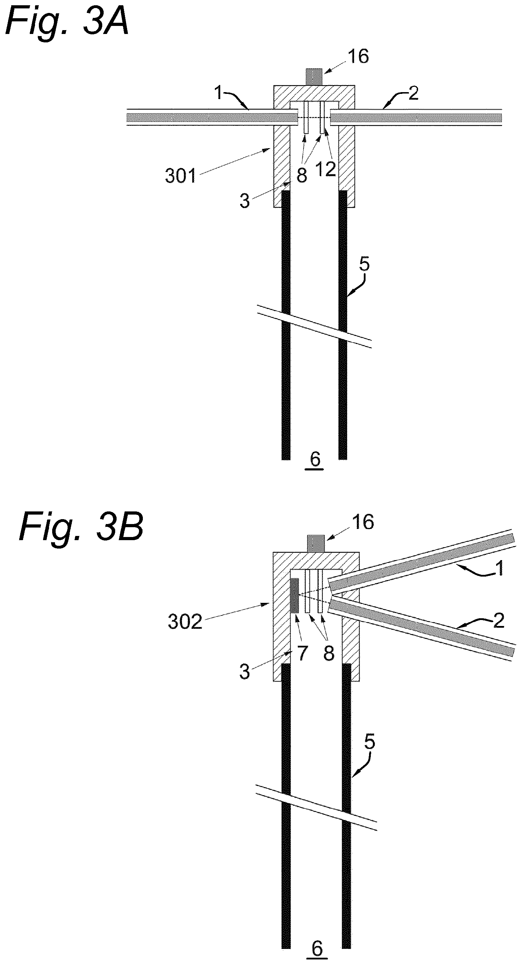

Fig. 3 illustrates a second example of a vapor density or surface condensation measurement probe. In this example, the measurement chamber is optimized for the detection of surface condensation. Fig. 3A shows a measurement probe 301 comprising a measurement chamber 3 with transmission geometry, whereas Fig. 3B shows a measurement probe 302 comprising a measurement chamber with reflection geometry. The configurations are highly similar to the configurations of Fig. 1A and Fig. 1B. Also, the measurement chamber of Fig. 3A or 3B can be implemented in the measurement device depicted in Fig. 2. Therefore, in the following, only the differences will be described in detail. In the arrangements of the second example, the light that enters the measurement chamber 3 via the first optical fiber 1 has to pass some additional sheets or lenses 8, which may be transparent, but provide surfaces along the optical path on which condensation may occur, before the light reaches the second optical fiber 2. Therefore, in such an arrangement the optical absorption will greatly depend on the presence of surface condensation on these additional sheets or lenses 8. The presence of condensation may be detected by means of a relatively simple algorithm, which may be based on a threshold value of the detected light intensity. For example, if the intensity of the light detected by the detector 206 is above a predetermined threshold, "no condensation" may be decided, whereas, if the intensity of the light detected by the detector 206 is below the predetermined threshold, "condensation" may be decided. Other detection criteria may also be applied.

The sheets or lenses 8 may be made of a material which is resistant to saturated steam at elevated temperatures (i.e., temperatures in the applied temperature range, for example between 100 and 150 degrees Celsius), is optionally impermeable to gases and fluids, optionally has a thermal conductivity that is substantially higher than that of an air-vapor mixture and is optically transparent (or, alternatively, reflective) for the wavelength(s) used for the probe. One of the materials that can be used is N-BK7 glass. Other materials are possible, but these may be less cost-effective in many cases.

For the condensation detector, it may be best if the temperature of the measurement chamber follows the temperature of the steam in the sterilizer chamber. This may be realized by not using a temperature control (heater), and placing the measurement probe within the sterilizer chamber. This way, it may be detected whether condensation occurs in the sterilizer chamber under the actual temperature and pressure conditions in the sterilizer chamber.

By combining the measurement by this device with a temperature measurement, for instance, by using a temperature sensor 16, the data will indicate in real-time whether sterilization conditions are present or not. Indeed, sterilization conditions may be decided to be present if condensation occurs *and* the temperature is above a predetermined threshold.

It will be understood that the additional sheets or lenses 8 are optional. For example, in the reflection geometry, illustrated in Fig. 3B, the reflective surface 7 can replace the sheet/lens. When condensation occurs on the reflective surface 7, the properties of the reflected light will change considerably, similar to the change in the transmission of light through the additional sheets or lenses 8. This change of the reflected light will be detected by the detector 260, so that the presence of the condensation on the reflective surface can be detected.

Similarly, in the transmission geometry shown in Fig. 3A, it is possible to detect the condensation without the additional sheets or lenses 8. In such a case, for example, condensation, which may form on the surface 103 of the tip of the optical fiber, where the optical fiber ends in the measurement chamber, may be detected. That surface 103 may be the surface of an interface between the light source and the inside of the measurement chamber, or the surface of the interface between the light detector and the inside of the measurement chamber. That "interface" may be the tip of the optical fiber itself, or for example a lens or a transparent plate through which the light flows into the measurement chamber. For example, in an implementation without an optical fiber this interface may be the separation between the lamp or sensor and the measurement chamber.

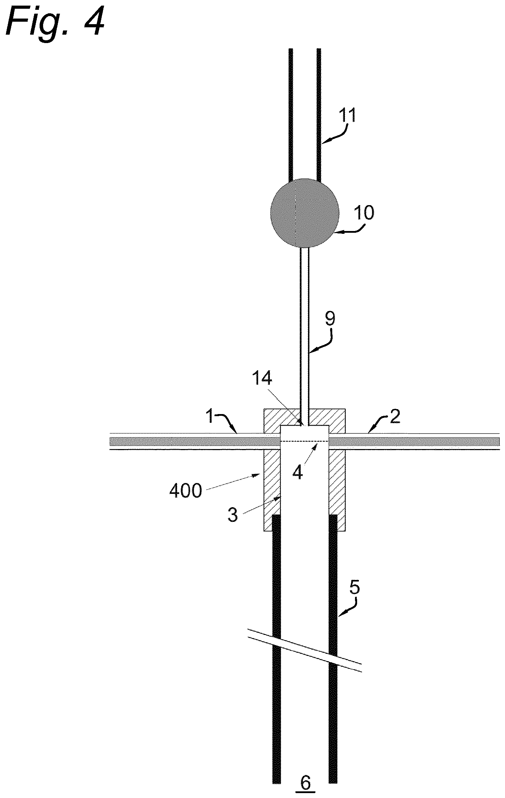

Fig. 4 illustrates a third example arrangement of the measurement probe. Although the arrangement is only illustrated in transmission geometry, the same principle can be applied to reflection geometry. Most of the elements of the arrangement are similar to the arrangement of the first example. Only the differences will be described hereinafter. In particular, the arrangement of the third example comprises a capillary tube 9 (which may be a tube of small internal diameter, for instance, 0.1 mm), a tube 11, and a valve 10 to fluidly connect the tube 11 to the capillary tube 9. After a typical sterilization process, some water vapor or condense will remain in the tube of a Process Challenge Device. Optionally, to improve the accuracy of the readings of the water vapor density, the measurement chamber and tube can be flushed after each process. This can be realized, for instance, by fluidly connecting the capillary tube 9 to the measurement chamber. The measurement chamber 4 and tube 5 can be flushed via the capillary tube 9 that is connected via a valve 10 to a tube 11 through which, e.g., dry air is supplied. The dry air may be supplied to the tube 11 by a pump (not shown), for example.

Although not illustrated, the capillary tube may alternatively be added to the arrangement of the second example, by adding the additional sheets or lenses 8 of the second example to the measurement chamber shown in Fig. 4.

This capillary tube can also be used in the calibration (or conditioning) of the sensor to obtain the above mentioned calibration constant $C_{cal}$. For this purpose the capillary tube can be connected to a source of saturated steam. Using a continuous flow of steam the capillary tube and the measurement chamber (Fig. 4, numeral 4) are filled. Sufficient measurements are performed while the measurement chamber 4 is filled with the saturated steam provided through the capillary tube 9 to obtain an accurate value of $C_{cal}$.

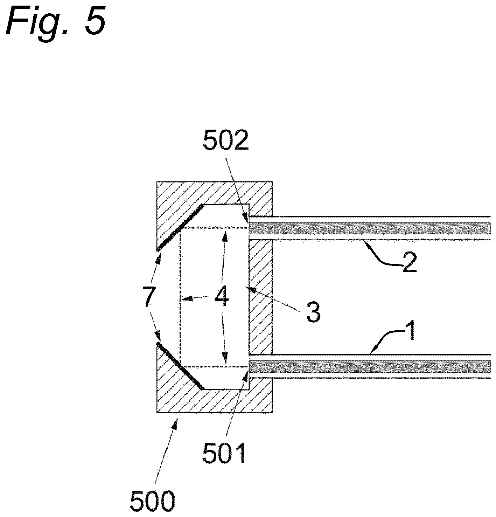

Fig. 5 illustrates a fourth example of an arrangement of a measurement probe. In the fourth example, the Process Challenge Device is omitted. Also, a reflective geometry is implemented using two reflective surfaces 7 (or mirrors). The optical path 4 through the measurement chamber 3 of Fig. 5 can be relatively long by using the extra reflective surfaces 7. In general, more reflective surfaces 7 may be added to further increase the length of the optical path 4 through the measurement chamber. For direct measurements of steam density in any environment, in particular within a sterilizer chamber, an arrangement like the one depicted in Fig. 5 can be used. The (optionally concave) reflective surfaces or mirrors 7 guide the light from one optical fiber 1 to the other optical fiber 2. Also in this configuration a lens may be positioned in the optical path. The measurement chamber 3 can be fitted to an object that is at a sufficiently high temperature to avoid condensation on the reflective surfaces 7, and/or the terminal surfaces 501, 502 of the optical fibers 1, 2. In many implementations, the wall of the sterilizer chamber is a suitable example of such a sufficiently heated object.

It will be understood that, similar to the reflective geometry configuration of Fig. 5, the transmission geometry configuration of Fig. 1 can also be implemented without the resistance 5. Moreover, it will be understood that a heating element 15 may be added to any of the configurations disclosed herein, in particular the configurations shown in Figs. 1A, 1B, 4, and 5. Since the configuration of Fig. 3 is aimed at detecting condensation, the heating element may be omitted in the configuration of Fig. 3. Also, a dry air supply, such as the one shown in Fig. 4, can be added to the configuration of Fig. 3A or 3B, so that any condensation in the measurement chamber can be blown away after and/or before the condensation detection. The arrangement in Fig. 5 (without heater) can also be used in a load, for example to detect the presence of surface condensation in a load. This load could comprise a pack of textile, for example.

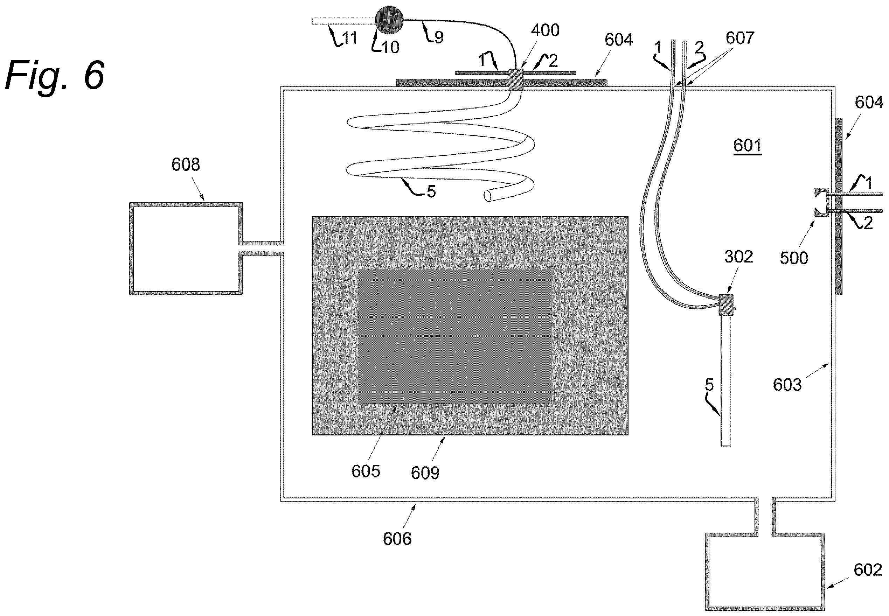

Fig. 6 shows a schematic representation of a sterilizer apparatus. Only features relevant to an understanding of the present disclosure have been illustrated in Fig. 6. Features not necessary for an understanding of the measurement probe have been omitted. The sterilizer apparatus comprises walls 606 forming an at least substantially closable sterilizer chamber 601. The sterilizer chamber 601 may have a loadable space 609, in which load 605 may be put. For example, at least one of the walls 606 may comprise a closable opening for inserting and removing the load 605 into and out of the sterilizer chamber 601. This load 605 may comprise for example a pack of textile and/or one or more medical devices to be sterilized. Such medical devices may include long, narrow tubes having non-water permeable walls.

The sterilizer chamber 601 may be fluidly connected to a pump 602. When the sterilizer chamber 601 is closed, the pump 602 may be optionally be configured to perform a pumping operation to remove any fluid from the sterilizer chamber 601 to create a vacuum inside the sterilizer chamber 601. The sterilizer may also comprise a steam generator 608 including a water supply and facilities to vaporize and heat the water. The steam may be conditioned, so that steam that is injected into the sterilizer chamber 601 may have predetermined properties including for example a predetermined temperature and/or a predetermined humidity. The steam generator 608 may comprise an electrical or other type of heating element to heat and vaporize the water. Alternatively, the steam may be supplied from an external source, such as a central heat generator of a hospital.

A typical sterilization process comprises three phases. The first phase is the conditioning phase, during which the air that is initially present in the sterilizer chamber is removed and the load is heated up to the sterilization temperature. This is generally achieved by successive cycles of evacuating the chamber by the pump 601 and injecting saturated steam from the steam generator 608. The second phase is the actual sterilization phase, during which the sterilizer chamber is filled with saturated steam and kept at the specified temperature (generally by controlling the pressure) for a specified time. During the third phase the sterilizer chamber is evacuated to dry the load and finally filled with air to atmospheric pressure to return to a safe state where it can be opened. Notwithstanding the above-disclosed typical sterilization process, alternative processes may be used to achieve the steam sterilization. The measurement probes disclosed herein may be used in conjunction with any suitable sterilization process.

One or more measurement probes can be used to monitor the sterilizing conditions inside the sterilizer chamber 601. However, the measurement probes disclosed herein may also be used in other measurement applications.

Fig. 6 illustrates three examples of how a measurement probe according to the present disclosure may be arranged with respect to the sterilizer chamber 601. However, these arrangements are only disclosed by means of examples, without limiting the present disclosure thereto.

In the first example, the measurement probe 302 may be "freely movable" within the sterilizer chamber 601. For example, it may be made possible to move the measurement probe 302 around in at least part of the loading space 609. For example, this may allow the user to put the measurement probe 302 within a load 605. This may be enabled by using a flexible cable, comprising for example flexible optical fibers 1 and 2, to connect the probe 302 to the orifices 607 in the wall of the sterilizer chamber 601. The light guides 1 and 2 of the light transmitter 210 and the light receiver 211, respectively, pass through respective orifices 607 in the wall 606 of the sterilizer chamber towards the lamp 202 and the light detector 206, respectively. Alternatively, the light guides 1 and 2 may also pass together through a single orifice. Yet alternatively, the lamp 202 and the light detector 206 may be located in the sterilizer chamber 601 or in the wall 606 of the sterilizer chamber 601. Since the light guides 1 and 2 are flexible, user may move around the probe 302 within the sterilizer chamber 601. For example, the probe 302 may be placed close to or in the middle of the load 605. In this example, the resistance 5 is a straight tube. However, this may be any kind of resistance. For example, a tube of an existing minimal invasive surgery (MIS) device may be replicated to be used as a representative resistance. Although the probe 302 (for condensation detection), which is shown in the drawing, is particularly suitable to be positioned anywhere in the sterilizer chamber 601, any of the other probes disclosed herein may be made freely movable in this way. When used for vapor density detection, the probe may be provided with a heating element 15 to avoid condensation, as described elsewhere in this disclosure. Also, the probe may be provided with a capillary tube 9 to flush any vapor and/or condensation out of the measurement chamber. The capillary tube 9 may, in that case, pass through the orifice or one of the orifices 607 in the wall 606 of the sterilizer chamber.

In the second example, the measurement probe 400 is fitted to the wall 606 of the sterilizer chamber 601. One side of the measurement probe 400 is inside the sterilizer chamber 601, whereas the other side of the measurement probe 400 is outside the sterilizer chamber 601. One end of the resistance 5 is fitted to the measurement probe 400 inside the sterilizer chamber 601, and fluidly connects the resistance 5 to the measurement chamber 3. The other end of the resistance 5 is open. The whole resistance 5 is inside the sterilizer chamber. Thus, the conditions of the resistance are similar to the conditions to which the load 605 is exposed. For example, a heating element 604 may be used to heat the wall 606 of the sterilizer chamber 601. Thus, the measurement probe 400, which is fitted to the wall 606, is heated also. Light guide 1 and 2 of probe 400 are provided, as described in detail elsewhere. A capillary tube 9, valve 10, and tube 11 may optionally be provided to flush the measurement chamber of the measurement probe 400 with a dry gas.

In the third example, the measurement probe 500 is fitted to the wall 606 of the sterilizer chamber, close to the heating element 604 of the sterilizer chamber wall 606. This way, the measurement chamber of the measurement probe 500 is kept to above a predetermined temperature. The measurement probe 500 is not attached to a Process Challenge Device or resistance. Consequently, the measurement performed by the measurement probe 500 is related to the general vapor conditions within the sterilizer chamber 601 rather than the specific condition inside a long tube disposed in the sterilizer chamber 601.

In any of the above arrangements, a controller 212 may be provided. The controller 212 may comprise one or more control devices that each may have their own functionality. These control devices may be implemented by means of semiconductor devices, for example. For example, one control device may control the operation of the sterilizer chamber 601 including injection of steam, whereas another control device may operate the measurement probe and generate a measurement output. However, this division of functionality is only an example. The control tasks disclosed herein may also be implemented in a single control device.

The controller may be configured to control operation of the LEDs 201, 202, and the ADC 207. It may also control the valve 10 connecting the tube 11 to the capillary tube 9. The controller may be responsible for various tasks.

For example, the controller may be configured to provide electricity to the LEDs to cause them to generate light at the desired times. Also, the controller may be configured to receive the values generated by the ADC. The controller may be configured to compute a vapor density based on the above-described equations. The controller may also be configured to detect formation of condensation, as described above, based on the values received from the ADC and/or a predetermined threshold value. The controller may be configured to generate an alarm signal if no condensation is detected in a preconfigured time interval. The controller may also be configured to adapt dynamically, for instance, the timing of the sterilization process based on the measurement result and/or the alarm signal.

In a particular embodiment, a first control device controls the sterilizer apparatus and a second control device controls the measurement probe. The first control device and the second control device may be configured to transmit and receive signals to and from each other, so that the measurements may be performed at an appropriate timing, and the sterilization process may be adjusted or optimized based on the measurements.

The controller may be configured to provide real-time information about the measured values and/or computed values. Also, the controller may be configured to detect the steam density and/or condensation formation during every sterilizing procedure.

Also, the controller may be configured to deliver at least some of the detected and/or computed results to a hospital information system. In such a system, the digital information may be coupled to patient files. Also, the digital information can be coupled to a relevant instrument, to track and trace systems. This relevant instrument can be the sterilizer device. Alternatively, digital information can be coupled to the device that was sterilized. Also, the digital information can be coupled to a maintenance system associated with the sterilizer device. For example, if malfunctioning is detected, the system can send a signal to a maintenance service, so that service may be provided to repair the sterilizer device. Additionally, the information of the sterilization process used for medical devices can be coupled to patient files in order to improve patient safety.

It will be understood that, although in the above disclosed examples, the light is guided to and from the measurement chamber 3 by means of optical fibers 1 and 2, these optical fibers may be replaced by other components. For example, instead of the first optical fiber 1, a lamp may be provided inside the measurement chamber or in the wall of the measurement chamber, wherein the lamp is configured in such a way that it radiates light of the prescribed wavelength(s) into the measurement chamber. The lamp may comprise, for example a LED. Two or more lamps may be provided, which may optionally be configured to radiate light with different wavelengths. Instead of the second optical fiber 2, a light detector may be provided inside the measurement chamber 3 or in the wall of the measurement chamber, wherein the detector is configured to detect light inside the measurement chamber 3. Alternatively, a broad band light source may be provided, combined with a spectrometer to analyze the spectral distribution of the detected light. The emission and detection, and the processing of the detected light intensities, is similar to the above-disclosed implementations that make use of the optical fiber.

The devices and methods disclosed herein allow monitoring variables that are relevant for determining steam sterilization conditions. They allow monitoring steam penetration for every load of the sterilizer. Unlike chemical and biological based tests, the disclosed device and method does not need subjective human interpretation to assess the real sterilizing conditions. The device can provide the measurement information in real-time. Also, it can be used to obtain more insight in the steam sterilization processes. Also, it allows conditioning the device before or during use.

As a more general application, the disclosed devices and methods can assess steam penetration in channels. The device can replace steam penetration tests as specified in the standards for steam sterilization [EN285, EN867-5, EN13060, ISO11140-3, ISO11140-4, ISO11140-5].

Since the presence of water vapor is measured directly by means of light measurements, it does not have to be inferred from indirect temperature (difference) measurements. The measurements can be performed inside the sterilizer chamber itself, directly probing the conditions of the loads to be sterilized. The use of optical fiber technology can help to make the system immune to electric or magnetic interference. Together with a temperature measurement, the system offers the possibility to monitor directly whether sterilization conditions are satisfied or not. The data of the measurements can be made available in real-time, so they can be used for process control or process optimization.

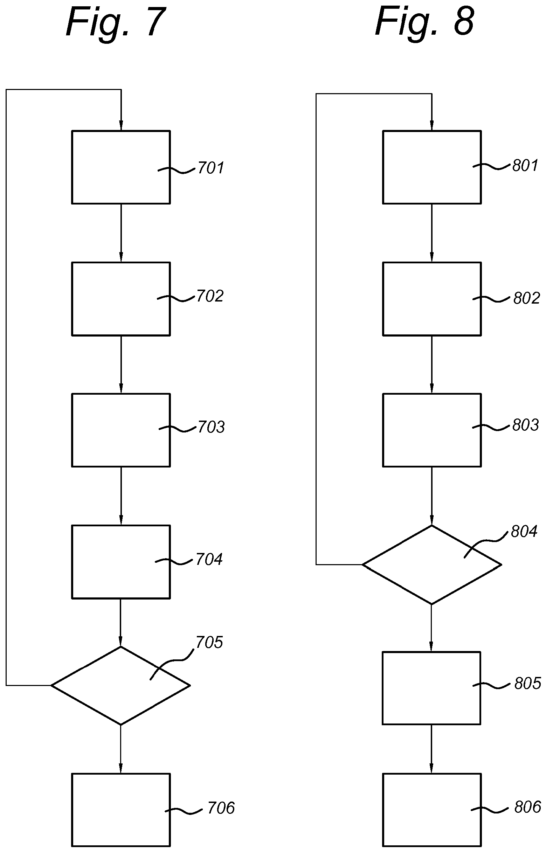

Fig. 7 illustrates a method of sterilizing a load. The method may start at step 701 of injecting steam into a sterilizer chamber having a fluid connection to a measurement chamber. This step may further comprise evacuating the sterilizer chamber and/or increasing the pressure of steam in the sterilizer chamber. The method further comprises step 702 of emitting light into a measurement chamber of a measurement probe, wherein the measurement chamber has a fluid connection to the sterilizer chamber. The method further comprises step 703 of detecting the light in the measurement chamber and converting the detected light into a detection signal. The method further comprises step 704 of heating at least part of the measurement probe to a temperature above a condensation temperature of the steam. This heating step may be performed such that at least a part of an inner wall of the measurement chamber is heated above the condensation temperature, so that no condensation can occur on that part of the inner wall. It will be understood that the method steps may be performed in any order, and in particular, may be performed in parallel. The heating step 704 may be performed at the same time as the steam injection step 701 and the steps of emitting light 702 and detecting the light 703 may be performed repeatedly while the steam is injected and/or the at least part of the measurement probe is heated. In step 705, it is decided whether the sterilizing process is finished. This decision may be based, for example, on the detection signal generated in step 703. If the sterilizing process is not yet finished, the process continues from step 701. If the sterilizing process is finished, the method proceeds at step 706 which represents finalizing steps that may include evacuating the steam from the sterilizer chamber, injecting dry gas, and creating atmospheric pressure inside the sterilizer chamber.

Fig. 8 illustrates another method of sterilizing a load. The method comprises step 801 of performing a sterilizing process to sterilize a load by injecting steam into a sterilizer chamber. This step may further comprise evacuating the sterilizer chamber and/or increasing the pressure of steam in the sterilizer chamber. The method further comprises step 802 of emitting light into a measurement chamber having a fluid connection to the sterilizer chamber and an orifice for supply of a gas. The method further comprises step 803 of detecting the light in the measurement chamber and converting the detected light into a detection signal. In step 804, it is decided whether the sterilizing process is finished. If the sterilizing process is not yet finished, the process continues from step 801 to continue the sterilizing process and optionally perform further measurements using the steps 802 and 803. It will be understood that, for example, the method steps 801, 802, and 803 may be performed in any order, and in particular, may be performed in parallel. The steps of emitting light 802 and detecting the light 803 may be performed repeatedly while the steam is injected and/or optionally the measurement probe is heated. In step 804, it is decided whether the sterilizing process is finished. This decision may be based, for example, on the detection signal generated in step 803. If the sterilizing process is not yet finished, the process continues from step 801. If the sterilizing process is finished, the method proceeds at step 805 which represents finalizing steps that may include evacuating the steam from the sterilizer chamber, injecting dry gas, and creating atmospheric pressure inside the sterilizer chamber. After step 805, or as part of step 805, the method proceeds to step 806 of blowing the gas through the orifice into the measurement chamber. This way, the measurement chamber may be flushed after the sterilization. In an alternative embodiment, the step 806 is performed before step 801.

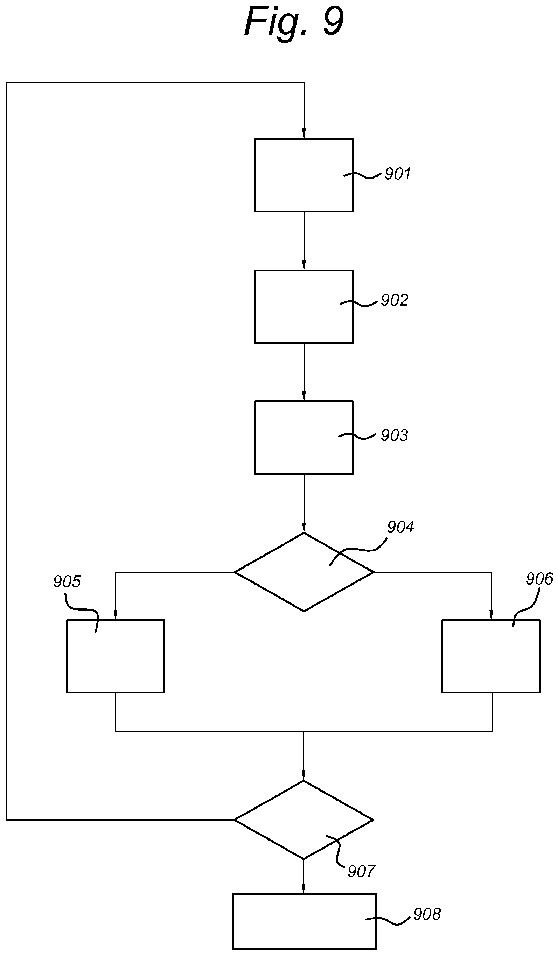

Fig. 9 illustrates a method of detecting condensation. The method comprises step 902 of emitting light onto or through a surface within a measurement chamber of a measurement probe. The method further comprises step 903 of detecting the light that has interacted with the surface and converting the detected light into a detection signal. The method further comprises step 904 of determining whether a liquid is detected on the surface based on the detection signal. For example, the detection signal is compared to a threshold value to decide whether the liquid is detected.

Optionally, the method comprises further steps to perform a sterilizing process. In that case, the method may start with a step 901 of injecting steam into a sterilizer chamber to sterilize an object within the sterilizer chamber, wherein the measurement chamber is fluidly connected to the sterilizer chamber. This step 901 may further comprise evacuating the sterilizer chamber and/or increasing the pressure of steam in the sterilizer chamber. It will be understood that the method steps may be performed in any order, and in particular, may be performed in parallel. The steps of emitting light 902, detecting the light 903, and detecting the liquid 904 may be performed repeatedly while the steam is injected according to step 901. If the liquid is detected in step 904, the process continues at step 905 which may comprise appropriate control actions of the sterilizing process, for example maintaining a timer of the total time that the liquid has been present. If the liquid is not detected in step 904, the process continues at step 906 which may comprise appropriate control actions of the sterilizing process, for example generating an alarm if the liquid is not detected for too long a time during the sterilization process.

In step 907, it is decided whether the sterilizing process is finished. This decision may be based, for example, on the detection signal generated in step 903 and the decision made in step 904 and action made in steps 905 and/or 906. If the sterilizing process is not yet finished, the process continues from step 901. If the sterilizing process is finished, the method proceeds at step 908 which represents finalizing steps that may include evacuating the steam from the sterilizer chamber, injecting dry gas, and creating atmospheric pressure inside the sterilizer chamber. Step 908 may also comprise blowing a (dry) gas through an orifice into the measurement chamber to flush the measurement chamber after the sterilization.

According to an example, an apparatus for detecting condensation is provided. The apparatus comprises

> a measurement probe comprising a measurement chamber comprising a surface;
> a light source configured to emit light onto or through the surface; and
> a light detector configured to detect the light that has interacted with the surface and convert the detected light into a detection signal.

The measurement probe is particularly suited to detect a condensation, because the light that has interacted with the surface will depend greatly on whether condensation has formed on the surface.

The light emitted onto or through the surface interacts with the surface, for example by transmission through the surface or by being reflected by the surface. After this interaction, at least part of the light is detected by the light detector. Thus, the light detector is placed such that at least part of the light that has interacted with the surface reaches the light detector. Thus, an optical path from the light source to the light detector via the surface is created within the measurement chamber.

The apparatus may further comprise a controller configured to determine whether a liquid is detected on the surface based on the detection signal and generate an output signal comprising an indication of whether the liquid is detected on the surface. Such a controller automates the interpretation of the detection signal. This is useful for many applications including but not limited to generating an alarm based on the indication, or (automatically) controlling a process based on the condensation measurement.

The detection signal may be indicative of an intensity of the detected light and the controller may be configured to determine whether the liquid is detected on the surface based on the intensity of the detected light. Intensity of the detected light may be greatly dependent on the presence of condensation on the surface, because the absorption or diffusion characteristics of the surface alter when condensation is formed on the surface.

The controller may be configured to control the light source to emit the light or control the light detector to detect the light. The light emitting and detection may be controlled by the control unit.

The surface may be planar or convex. Alternatively, least part of the surface may be planar or convex. This is a particularly suitable shape of the surface. Planar surface may allow the light to transmit through the surface substantially undistorted, whereas the convex surface may be used to create a lens effect, for example to direct more of the light towards the light detector.

The apparatus may comprise a sheet or lens, at least part of at least one side of the sheet or lens forming the surface. The sheet or lens may be a particularly suitable implementation of the surface.

For example, at least part of the sheet or lens may be transparent or reflective with respect to the light emitted by the light source. This way, the lens can act as a glass or a mirror.

For example, both sides of the sheet or lens may be in direct contact with the gas inside the measurement chamber. Since both sides of the sheet or lens contact the gas in the measurement chamber, the contacting surface is particularly large. Also, the temperature of the sheet or lens may be more similar to the temperature of the gas in the measurement chamber. In a particular implementation example, the sheet or lens may be fixed to a wall on one side of the sheet or lens and protrude into the measurement chamber from the wall of the measurement chamber.

In a particular example, an interface between the light source or light detector and an inside of the measurement chamber comprises the surface. This is a relatively simple implementation in which the condensation on the surface on the interface is detected.

The apparatus may comprise a resistance, such as a tube, fitted to the measurement probe, wherein a first end of the resistance has a fluid connection to the measurement chamber and a second end of the resistance is open. This tube can act as a 'process challenge device', to replicate the conditions inside other tubes in the same environment as the apparatus. For example, it can be used to assess whether condensation occurs in such a tube.

The apparatus may comprise a steam sterilizer comprising a sterilizer chamber, wherein the steam sterilizer is configured to inject steam into the sterilizer chamber to sterilize an object within the sterilizer chamber; wherein the measurement chamber has a fluid connection to the sterilizer chamber. The fluid connection of the measurement chamber to the sterilizer chamber allows to assess the steam conditions inside the sterilizer chamber. The steam sterilizer may further be configured to evacuate the sterilizer chamber before injecting the steam or to perform evacuation of sterilizer chamber and injection of the steam alternatingly.

The measurement probe may be freely movable within at least a loading space of the sterilizer chamber. For example, in some embodiments, the measurement probe is not rigidly fixed inside the sterilizer chamber but may be moved around. The measurement probe may optionally still be attached to the sterilizer chamber by means of, for example, a flexible cable.

The measurement chamber may have a fluid connection to the sterilizer chamber through a resistance, such as a tube. This helps to create a process challenge device to assess the sterilization conditions with regards to the sterilization of the inside of a tubular instrument.

The sterilizer chamber may comprise the measurement probe. Thus, the measurement probe may be fixed to the inside of the sterilizer chamber. This may be done by rigidly attaching the measurement probe directly to e.g. a wall of the sterilizer chamber, or by attaching the measurement probe via a flexible cable to the inside of the sterilizer chamber.

The apparatus may comprise a controller configured to, repeatedly during a sterilization process performed by the steam sterilizer, receive the detection signal from the light detector and determine whether the liquid is detected on the surface based on the detection signal. This is helpful to assess during what time period or which time periods the sterilization conditions are met. For example, sterilization standards may prescribe a certain period of time during which condensation should form on the surfaces that have to be sterilized. By performing the detection and analyzing the detection signal repeatedly during an interval, it can be assessed whether the prescribed period has been reached.

The steam sterilizer may be configured to adjust the sterilization process based on whether the liquid is detected on the surface during the sterilization process. For example, if the condensation was first detected at a given time period t, then the steam sterilizer may be configured to continue its sterilization process for at least a predetermined time interval Δt, up to t+Δt. Also, if absence of condensation is detected at a time at which condensation was expected to occur, the sterilizer may be configured to adjust the process for example by increasing the supply of steam. Other manners of adjusting the process based on whether the liquid is detected may also be applied.

The condensation may comprise condensation of water vapor. This is particularly suitable for a steam sterilizer apparatus to monitor the sterilization process. The control unit may detect water vapor condensation based on the detected light intensities. The frequency of emitted light may be selected so that the light transmitted through or reflected by the surface greatly depends on the presence of condensation.

The light source may comprise at least one of a lamp and a light guide. The light source may comprise both a lamp and a light guide. The light guide may be configured to guide the light from the lamp to the measurement chamber and towards the surface.

The light detector may comprise at least one of a light sensor and a light guide. The light detector may comprise both the light sensor and the light guide. The light guide may be configured to guide the light reflected from or transmitted through the surface towards and onto the light sensor. To this end, the light guides of the light source and light detector are positioned so that these light guides cooperate with each other to form a light path through the measurement chamber from the light source via the surface to the light detector.

According to another example, a method of detecting condensation is provided. The method comprises

emitting light onto or through a surface within a measurement chamber of a measurement probe;
detecting the light that has interacted with the surface and converting the detected light into a detection signal; and

determining whether a liquid is detected on the surface based on the detection signal.

This method is highly suitable to monitor the vapor condition.

The method may comprise injecting steam into a sterilizer chamber to sterilize an object within the sterilizer chamber, wherein the measurement chamber is fluidly connected to the sterilizer chamber. The presence of condensation is an important factor of successful sterilization.

According to an example, the device and method incorporate an optical setup which can measure the water vapor density or detect the presence of surface condensation. This setup can either be placed directly in a sterilizer chamber or embedded in a measurement chamber that can be connected to a Process Challenge Device, for instance a long tube. The device can also be used as a parameter input to control the steam sterilization process.

According to an example, a sterilizer apparatus is provided. The sterilizer apparatus comprises

a sterilizer chamber, wherein the sterilizer apparatus is configured to inject steam into the sterilizer chamber;
a measurement probe comprising a measurement chamber having a fluid connection to the sterilizer chamber;
a light source configured to emit light into the measurement chamber;
a light detector configured to detect the light in the measurement chamber and convert the detected light into a detection signal; and
a heating element configured to heat at least part of the measurement probe to a temperature above a condensation temperature of the steam.

The heating element avoids that condensation is formed in the measurement chamber. This way, a measurement can be performed on the non-condensed steam inside the measurement chamber using the light source and light detector. In a particular example, the steam sterilizer may further be configured to evacuate the sterilizer chamber before injecting the steam or to perform evacuation of sterilizer chamber and injection of the steam alternatingly.

The heating element may be shared with a wall of the sterilization chamber, so that the heating element is configured to heat both at least part of the measurement probe and at least part of the wall of the sterilization chamber up to above said condensation temperature. This is an efficient implementation, as a single heating element may be used for two functions at the same time: heating the wall of the sterilization chamber and heating the measurement probe. For example, the heating element may be fixed to or embedded in the wall of the sterilizer chamber. In addition or alternatively, the measurement probe may be fixed to the wall of the sterilizer chamber.

The fluid connection from the measurement chamber to the sterilizer chamber may comprise a resistance such as a tube. This allows to replicate conditions of tubular instruments inside the measurement chamber. Thus, the measurement can be used to assess a condition inside such tubular instruments. For example, a first end of the resistance may be fixed to the measurement chamber and a second end of the resistance is movable. This way, the second end of the resistance may be put somewhere among the load, e.g. somewhere in a stack of tubular instruments that are to be sterilized.

The sterilizer apparatus may be configured to perform a sterilizing process to sterilize a load while controlling the heating element to heat said at least part of the measurement probe to said temperature above the condensation temperature of the steam. Doing the heating of the measurement probe during the sterilization process allows performing the measurements using the light source and light detector reliably during the sterilization process.

The sterilizer apparatus may comprise a controller configured to determine an indication of a vapor density in the measurement chamber based on the detection signal. This vapor density can be determined more reliably if condensation in the measurement chamber is avoided.

The controller may be configured to, repeatedly during a sterilization process performed by the sterilizer apparatus, receive the detection signal from the light detector and determine the indication of the vapor density or whether the liquid is present based on the detection signal. This way, the changes in vapor density during the sterilization process may be monitored, to assess sterilization quality.

The sterilizer apparatus may be configured to adjust the sterilization process based on the indication of the vapor density. This adjustment may be performed under control of a controller or processor, for example. The adjustment allows to guarantee a minimum sterilization quality by adjusting the sterilization process until the minimum desired sterilization condition has been realized and measured by the measurement probe.

The vapor may comprise water vapor. This makes the apparatus highly suitable for processes involving steam sterilization which use water vapor to sterilize certain materials.

The light source may comprise at least one of a lamp and a light guide. The light source may comprise both a lamp and a light guide. The light guide may be configured to guide the light from the light source to the measurement chamber and towards the surface.

The light detector may comprise at least one of a light sensor and a light guide. The light detector may comprise both the light sensor and the light guide. The light guide may be configured to guide the light reflected from or transmitted through the surface towards and onto the light sensor. To this end, the light guides of the light source and light detector are positioned so that these light guides cooperate with each other to form a light path through the measurement chamber from the light source via the surface to the light detector.

The sterilizer chamber may comprise the measurement probe. That is, the measurement probe or at least the measurement chamber of the measurement probe may be located inside the sterilizer chamber. This allows the measurement probe to measure the conditions inside the sterilizer chamber.

According to an example, a method of sterilizing a load is provided. The method comprises

injecting steam into a sterilizer chamber having a fluid connection to a sterilizer chamber;

emitting light into a measurement chamber of a measurement probe, wherein the measurement chamber has a fluid connection to the sterilizer chamber;

detecting the light in the measurement chamber and converting the detected light into a detection signal; and

heating at least part of the measurement probe to a temperature above a condensation temperature of the steam.

The present invention has been described above with reference to a number of exemplary embodiments as shown in the drawings. Modifications and alternative implementations of some parts or elements are possible. The scope of the invention is defined by the appended claims, anything else is for information only.

Some or all aspects of the invention may be suitable for being implemented in form of software, in particular a computer program product. The computer program product may comprise a computer program stored on a non-transitory computer-readable media. Also, the computer program may be represented by a signal, such as an optic signal or an electro-magnetic signal, carried by a transmission medium such as an optic fiber cable or the air. The computer program may partly or entirely have the form of source code, object code, or pseudo code, suitable for being executed by a computer system. For example, the code may be executable by one or more controllers or processors. Such controllers and processors, capable of executing a program and controlling a device, are known in the art.

The examples and embodiments described herein serve to illustrate rather than limit the invention. The scope of the invention is defined in the appended claims.

References

| Label | Reference |
|---|---|
| [BEN11] | Benoit F, Merger D, Hermsen RJ, and van Doornmalen JPCM. A comparison of four commercially available electronic steam penetration tests according to ISO 11140 part 4. Central Service, 3: 180-184, 2011. |
| [B&D63] | Bowie JH, Kelsey JC, and Thompson GR. The Bowie and Dick autoclave tape test. The Lancet, 281:568-569, 1963. |
| [ESEN11-1] | Esen S and van Doornmalen JPCM. One set of requirements for steam penetration tests is enough. Central Service, 5:365-367, 2011. |
| [ESEN12-1] | Esen S, Tessarolo F, Hermsen RJ, and van Doornmalen JPCM. Current reference devices for hollow instrument loads as defined in standards are not a valid steam penetration test. Central Service, 4: 256-260, 2012. |
| [EN 285] | European Committee for Standardization. Standard EN 285: A2:2009 sterilization - steam sterilizers - large sterilizers, 2015. |
| [EN867-5] | European Committee for Standardization. Standard EN 867-5, Non-biological systems for use in sterilizers. Specification for indicator systems and process challenge devices for use in performance testing for small sterilizers Type B and Type S, 2001. |
| [EN13060] | European Committee for Standardization. Standard EN 13060, Small Steam Sterilizers. European standard, 2014. |
| [IAWPS] | Wagner W, Cooper JR, Dittmann A, Kijima J, Kretzschmar HJ, Kruse A, Mares R, Oguchi K, Sato H, Stöcker I, Sifner O, Takaishi Y, Tanishita I, and Trübenbach IJ. The IAWPS industrial formulation 1997 for the thermodynamic properties of water and steam. Transactions of the ASME, 122:150-182, 2000. |
| [ISO11140-3] | International Organization for Standardization. Standard ISO 11140-3Sterilization of health care products - Chemical indicators - Part 3: Class 2 indicators systems for use in a Bowie and Dick-type steam penetration. ISO standard, 2007. |

(continued)

| Label | Reference |
|---|---|
| [ISO11140-4] | International Organization for Standardization. Standard ISO 11140-4 Sterilization of health care products - Chemical indicators - Part 4: Class 2 indicators as an alternative to the Bowie and Dick-type test for detection of steam penetration. ISO standard, 2007. |
| [ISO11140-5] | International Organization for Standardization. Standard ISO 11140-5 Sterilization of health care products - Chemical indicators - Part 4: Class 2 indicators for Bowie and Dick-type air removal tests. ISO standard, 2007. |
| [ISO17665] | International Organisation for Standardisation. Standard ISO 17665-1. Sterilization of health care products -Moist heat, international organization for standardization, 2006. |
| [KIRK12] | Kirk B. An evaluation of nine Bowie and Dick test products available in the United Kingdom. Medical Device Decontamination 2012; 17(1): 8-23. |
| [MRC59] | Working party on Pressure Steam Sterilizers of the Medical Research Council. Sterilisation by steam under increased pressure. The Lancet, 273:425-435, 1959. |
| [SYK67] | Sykes G. Disinfection & sterilization. 2nd edition with corrections. E & FN Spon Ltd, London, 1967. |
| [VDO12-2] | van Doornmalen JPCM, Hermsen RJ, and Kopinga K. Six commercially available class 6 chemical indicators tested against their stated values. Central Service, 6:400-404, 2012. |
| [VDO13-1] | van Doornmalen JPCM, Verschueren M, and Kopinga K. Penetration of water vapour into narrow channels during steam sterilization processes. Journal of Physics D: Applied Physics, 46:065201, 2013.] |
| [VDO13-2] | van Doornmalen JPCM, Rietmeijer AGM, Feilzer AJ, and Kopinga K. Monitoring of steam sterilization processes in the dental office. Central Service, 6:435-438, 2013. |
| [VDO13-3] | van Doornmalen JPCM and Kopinga K. Measuring non-condensable gases in steam. Review of Scientific Instruments, 84:115106, 2013. |
| [VDO14-1] | van Doornmalen JPCM, Tessarolo F, and Kopinga K. Measurements of only pressure and temperature are insufficient to monitor steam sterilization processes: a case study. Central Service, 4:250-253, 2014. |
| [VDO15-1] | van Doornmalen JPCM, van Wezel RAC, and van Doornmalen HWJM. Case study on the orientation of phaco hand pieces during steam sterilization processes. Journal of Hospital Infection, 90:52-58, 2015. |
| [VDO15-2] | van Doornmalen JPCM and Kopinga K. Steam penetration in thin-walled channels and helix shaped process challenge devices. Central Service, 6:429-433, 2015. |

**Claims**

1. A sterilizer apparatus, configured to perform a sterilizing process to sterilize a load (605) and comprising

a sterilizer chamber (601), wherein the sterilizer apparatus is configured to inject steam into the sterilizer chamber (601);
a measurement probe (400) comprising a measurement chamber (3) having a fluid connection to the sterilizer chamber (601);
a light source (1) configured to emit light into the measurement chamber (3); and
a light detector (2) configured to detect the light in the measurement chamber (3) and convert the detected light into a detection signal;
wherein the measurement chamber (3) comprises an orifice (14) for supply of a dry gas, the orifice and the fluid connection with the sterilizer chamber being on opposite sides of the measurement chamber,

and wherein the sterilizer apparatus is configured to automatically blow the dry gas through the orifice (14) into the measurement chamber (3) before or after the sterilizing procedure.

**2.** The apparatus according to claim 1, wherein a capillary tube (9) is fitted to the orifice (14) to fluidly connect the measurement chamber (3) to a source of the dry gas.

**3.** The apparatus according to claim 2, comprising a valve (10) to selectively block or allow the passage of dry air from the source of the dry air via the capillary tube (9) into the measurement chamber (3).

**4.** The apparatus according to any of the preceding claims, wherein a controller (212) is configured to determine an indication of a vapor density or whether a liquid is present in the measurement chamber based on the detection signal.

**5.** The apparatus according to any of the preceding claims, wherein a controller (212) is configured to, repeatedly during a sterilization process performed by the sterilizer apparatus, receive the detection signal from the light detector (2) and determine the indication of the vapor density or whether the liquid is present based on the detection signal.

**6.** The apparatus according to claim 4 or 5, wherein the steam sterilizer is configured to adjust the sterilization process based on the indication of the vapor density or whether the liquid is present.

**7.** The apparatus according to any of the preceding claims, wherein the fluid connection to the sterilizer chamber comprises a tube (5), wherein a first end of the tube (5) has a fluid connection to the measurement chamber (3) and a second end of the tube is open.

**8.** A method of sterilizing a load (605), comprising

performing (801) a sterilizing process to sterilize a load by injecting steam into a sterilizer chamber;
emitting (802) light into a measurement chamber having a fluid connection to the sterilizer chamber and an orifice for supply of a dry gas, the orifice and the fluid connection with the sterilizer chamber being on opposite sides of the measurement chamber;
detecting (803) the light in the measurement chamber and converting the detected light into a detection signal; and
blowing (806) the dry gas through the orifice into the measurement chamber before or after the sterilizing procedure.

**Patentansprüche**

**1.** Sterilisatoreinrichtung, die konfiguriert ist, um einen Sterilisationsvorgang durchzuführen, um eine Ladung (605) zu sterilisieren, und Folgendes umfasst:

eine Sterilisatorkammer (601), wobei die Sterilisatoreinrichtung konfiguriert ist, um Dampf in die Sterilisatorkammer (601) einzuspritzen;
eine Messsonde (400), die eine Messkammer (3) umfasst, die eine Fluidverbindung mit der Sterilisatorkammer (601) aufweist;
eine Lichtquelle (1), die konfiguriert ist, um Licht in die Messkammer (3) zu emittieren; und
einen Lichterfasser (2), der konfiguriert ist, um das Licht in der Messkammer (3) zu erfassen und das erfasste Licht in ein Erfassungssignal umzuwandeln;
wobei die Messkammer (3) eine Öffnung (14) für eine Zufuhr eines Trockengases umfasst, wobei sich die Öffnung und die Fluidverbindung mit der Sterilisatorkammer auf gegenüberliegenden Seiten der Messkammer befinden,
und wobei die Sterilisatoreinrichtung konfiguriert ist, um das Trockengas durch die Öffnung (14) in die Messkammer (3) vor oder nach dem Sterilisationsablauf automatisch zu blasen.

**2.** Einrichtung nach Anspruch 1, wobei ein Kapillarrohr (9) an der Öffnung (14) angebracht ist, um die Messkammer (3) mit einer Quelle des Trockengases fluidisch zu verbinden.

**3.** Einrichtung nach Anspruch 2, die ein Ventil (10) umfasst, um den Durchgang von Trockenluft von der Quelle der Trockenluft über das Kapillarrohr (9) in die Messkammer (3) wahlweise zu blockieren oder zu ermöglichen.

**4.** Einrichtung nach einem der vorhergehenden Ansprüche, wobei eine Steuerung (212) konfiguriert ist, um eine Angabe einer Dampfdichte oder ob eine Flüssigkeit in der Messkammer vorhanden ist basierend auf dem Erfassungssignal zu bestimmen.

**5.** Einrichtung nach einem der vorhergehenden Ansprüche, wobei eine Steuerung (212) konfiguriert ist, um während eines Sterilisationsvorgangs, der mittels der Sterilisatoreinrichtung durchgeführt wird, das Erfassungssignal von dem Lichterfasser (2) wiederholt zu empfangen und die Angabe der Dampfdichte oder ob die Flüssigkeit basierend auf dem Erfassungssignal vorhanden ist, zu bestimmen.

**6.** Einrichtung nach Anspruch 4 oder 5, wobei der Dampfsterilisator konfiguriert ist, um den Sterilisationsvorgang basierend auf der Angabe der Dampfdichte oder ob die Flüssigkeit vorhanden ist, anzupassen.

**7.** Einrichtung nach einem der vorhergehenden Ansprüche, wobei die Fluidverbindung mit der Sterilisatorkammer ein Rohr (5) umfasst, wobei ein erstes Ende des Rohrs (5) eine Fluidverbindung mit der Messkammer (3) aufweist und ein zweites Ende des Rohrs offen ist.

**8.** Verfahren zum Sterilisieren einer Ladung (605), das Folgendes umfasst:

Durchführen (801) eines Sterilisationsvorgangs, um eine Ladung mittels Einspritzen von Dampf in eine Sterilisatorkammer zu sterilisieren;
Emittieren (802) von Licht in eine Messkammer, die eine Fluidverbindung mit der Sterilisatorkammer und eine Öffnung für die Zufuhr eines Trockengases aufweist, wobei sich die Öffnung und die Fluidverbindung mit der Sterilisatorkammer auf gegenüberliegenden Seiten der Messkammer befinden;
Erfassen (803) des Lichts in der Messkammer und Umwandeln des erfassten Lichts in ein Erfassungssignal; und
Blasen (806) des Trockengases durch die Öffnung in die Messkammer vor oder nach dem Sterilisationsablauf.

## Revendications

**1.** Appareil de stérilisation, conçu pour effectuer un processus de stérilisation afin de stériliser une charge (605) et comprenant

une chambre de stérilisation (601), l'appareil de stérilisation étant conçu pour injecter de la vapeur dans la chambre de stérilisation (601) ;
une sonde de mesure (400) comprenant une chambre de mesure (3) ayant un raccord de fluide à la chambre de stérilisation (601) ;
une source de lumière (1) conçue pour émettre de la lumière dans la chambre de mesure (3) ; et
un détecteur de lumière (2) conçu pour détecter la lumière dans la chambre de mesure (3) et convertir la lumière détectée en un signal de détection ;
la chambre de mesure (3) comprenant un orifice (14) pour l'alimentation d'un gaz sec, l'orifice et le raccord de fluide avec la chambre de stérilisation étant sur des côtés opposés de la chambre de mesure,
et l'appareil de stérilisation étant conçu pour souffler automatiquement le gaz sec à travers l'orifice (14) dans la chambre de mesure (3), avant ou après la procédure de stérilisation.

**2.** Appareil selon la revendication 1, dans lequel un tube capillaire (9) est ajusté sur l'orifice (14) pour raccorder de manière fluidique la chambre de mesure (3) à une source du gaz sec.

**3.** Appareil selon la revendication 2, comprenant une valve (10) pour bloquer ou permettre sélectivement le passage de l'air sec depuis la source de l'air sec par l'intermédiaire du tube capillaire (9) dans la chambre de mesure (3).

**4.** Appareil selon l'une quelconque des revendications précédentes, dans lequel un dispositif de commande (212) est configuré pour déterminer une indication d'une densité de vapeur ou le fait de savoir si un liquide est présent dans la chambre de mesure sur la base du signal de détection.

**5.** Appareil selon l'une quelconque des revendications précédentes, dans lequel un dispositif de commande (212) est configuré, de manière répétée pendant un processus de stérilisation effectué par l'appareil de stérilisation, pour recevoir le signal de détection du détecteur de lumière (2) et déterminer l'indication de la densité de vapeur ou le fait de savoir si le liquide est présent sur la base du signal de détection.

**6.** Appareil selon la revendication 4 ou 5, dans lequel le stérilisateur à vapeur est conçu pour ajuster le processus de stérilisation sur la base de l'indication de la densité de vapeur ou de la présence du liquide.

7. Appareil selon l'une quelconque des revendications précédentes, dans lequel le raccord de fluide à la chambre de stérilisation comprend un tube (5), une première extrémité du tube (5) ayant un raccord de fluide à la chambre de mesure (3) et une seconde extrémité du tube étant ouverte.

8. Procédé de stérilisation d'une charge (605), comprenant

le fait d'effectuer (801) un processus de stérilisation pour stériliser une charge en injectant de la vapeur dans une chambre de stérilisation ;
l'émission (802) de lumière dans une chambre de mesure ayant un raccord de fluide à la chambre de stérilisation et un orifice pour l'alimentation d'un gaz sec, l'orifice et le raccord de fluide avec la chambre de stérilisation étant sur des côtés opposés de la chambre de mesure ;
la détection (803) de la lumière dans la chambre de mesure et la conversion de la lumière détectée en un signal de détection ; et
le soufflage (806) du gaz sec à travers l'orifice dans la chambre de mesure, avant ou après la procédure de stérilisation.

## Fig. 1A

## Fig. 1B

Fig. 2

## Fig. 3A

## Fig. 3B

# Fig. 4

*Fig. 5*

Fig. 6

# Fig. 7

701

702

703

704

705

706

# Fig. 8

801

802

803

804

805

806

# Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5270217 A
- US 4115068 A
- DE 102010016017 A1
- DE 202006006926 U1
- EP 1230936 A1
- WO 201047139 A
- US 6323031 B
- DE 202014009495 U1
- US 4507875 A

**Non-patent literature cited in the description**

- **VAN DOORNMALEN ; GOMEZ HOYOS.** *Surface steam sterilization: Steam penetration in narrow channels,* ISBN 978-90-38-63442-5
- **BENOIT F ; MERGER D ; HERMSEN RJ ; VAN DOORNMALEN JPCM.** A comparison of four commercially available electronic steam penetration tests according to ISO 11140 part 4. *Central Service,* 2011, vol. 3, 180-184
- **BOWIE JH ; KELSEY JC ; THOMPSON GR.** The Bowie and Dick autoclave tape test. *The Lancet,* 1963, vol. 281, 568-569
- **ESEN S ; VAN DOORNMALEN JPCM.** One set of requirements for steam penetration tests is enough. *Central Service,* 2011, vol. 5, 365-367
- **ESEN S ; TESSAROLO F ; HERMSEN RJ ; VAN DOORNMALEN JPCM.** Current reference devices for hollow instrument loads as defined in standards are not a valid steam penetration test. *Central Service,* 2012, vol. 4, 256-260
- **WAGNER W ; COOPER JR ; DITTMANN A ; KIJIMA J ; KRETZSCHMAR HJ ; KRUSE A ; MARES R ; OGUCHI K ; SATO H ; STÖCKER I.** The IAWPS industrial formulation 1997 for the thermodynamic properties of water and steam. *Transactions of the ASME,* 2000, vol. 122, 150-182
- **KIRK B.** An evaluation of nine Bowie and Dick test products available in the United Kingdom. *Medical Device Decontamination,* 2012, vol. 17 (1), 8-23
- Working party on Pressure Steam Sterilizers of the Medical Research Council. Sterilisation by steam under increased pressure. *The Lancet,* 1959, vol. 273, 425-435
- **SYKES G.** Disinfection & sterilization. E & FN Spon Ltd, 1967
- **VAN DOORNMALEN JPCM ; HERMSEN RJ ; KOPINGA K.** Six commercially available class 6 chemical indicators tested against their stated values. *Central Service,* 2012, vol. 6, 400-404
- **VAN DOORNMALEN JPCM ; VERSCHUEREN M ; KOPINGA K.** Penetration of water vapour into narrow channels during steam sterilization processes. *Journal of Physics D: Applied Physics,* 2013, vol. 46, 065201
- **VAN DOORNMALEN JPCM ; RIETMEIJER AGM ; FEILZER AJ ; KOPINGA K.** Monitoring of steam sterilization processes in the dental office. *Central Service,* 2013, vol. 6, 435-438
- **VAN DOORNMALEN JPCM ; KOPINGA K.** Measuring non-condensable gases in steam. *Review of Scientific Instruments,* 2013, vol. 84, 115106
- **VAN DOORNMALEN JPCM ; TESSAROLO F ; KOPINGA K.** Measurements of only pressure and temperature are insufficient to monitor steam sterilization processes: a case study. *Central Service,* 2014, vol. 4, 250-253
- **VAN DOORNMALEN JPCM ; VAN WEZEL RAC ; VAN DOORNMALEN HWJM.** Case study on the orientation of phaco hand pieces during steam sterilization processes. *Journal of Hospital Infection,* 2015, vol. 90, 52-58
- **VAN DOORNMALEN JPCM ; KOPINGA K.** Steam penetration in thin-walled channels and helix shaped process challenge devices. *Central Service,* 2015, vol. 6, 429-433